# EUROPEAN PATENT SPECIFICATION

(11) **EP 3 355 290 B1**
(45) Date of publication and mention of the grant of the patent: **27.11.2019**
(21) Application number: 17158919.5
(22) Date of filing: 02.03.2017
(51) Int. Cl.: G08B 21/14, F24C 7/08

(54) **SYSTEM AND METHOD FOR PROTECTION OF A PERSON AGAINST CONTAMINATION OF AIR**
SYSTEM UND VERFAHREN ZUM SCHUTZ EINER PERSON GEGEN LUFTVERSCHMUTZUNG
SYSTÈME ET MÉTHODE DE PROTECTION D'UNE PERSONNE CONTRE LA CONTAMINATION DE L'AIR

(30) Priority: 26.01.2017 CN 201710057023
(43) Date of publication of application: 01.08.2018
(73) Proprietor: BSH Hausgeräte GmbH, 81739 München (DE)
(72) Inventor: ARTAL LAHOZ, Maria Carmen, 50007 Zaragoza (ES); CHU, Dongyu, Zhaozhuang, 277315 (CN); LI, Chao, Nanjing City, 210014 (CN); OERTEL, Kai, 573943 Singapore (SG); PALALE, Suresh, 57943 Singapore (SG); SCHLOTMANN, Peter, 49219 Glandorf (DE); SENGEBUSCH, Falco, 573943 Singapore (SG); SOON, Hwee Ping, 573943 Singapore (SG); VON BIEREN, Arndt, 81829 München (DE); YAN, Yan, 573943 Singapore (SG); YANG, Lei, Nanjing, 210000 (CN)

(56) References cited:
- EP-A1- 3 103 390
- WO-A1-2015/168243
- WO-A1-2016/123111

## Description

The present invention concerns a technique for ensuring health of a person in the vicinity of a home appliance for preparing food, especially a cooker.

When preparing food on a cooker, air in the vicinity of the cooker may get enriched with vapours or particles that spread odours. A cooking hood may be used to remove air from the area of the cooker. Usually, the hood is placed above the cooker and contains a ventilation system that sucks up air and air pollutants, filters the air and leads it to a different place. There are also designs that cause an air draught to the side or below a cooktop. Nevertheless, removal of cooking mist is rarely total and a person cooking food on the cooker may be exposed to unpleasant by-products.

EP 2 735 809 A1 proposes a cooker hood with an interface to a sensor for measuring air quality. If the air quality is bad, a ventilation system inside the cooker hood is controlled to remove air from the cooker at a higher rate.

US 2008 / 0 045 156 A1 discloses a personal air monitoring device. A person carrying the device can be warned if a concentration of a pollutant like CO₂ or ozone exceeds a predetermined level.

US 6 920 874 B1 concerns an intelligent ventilating safety range hood. Air convection through the hood is controlled on the basis of a measurement of air pollution inside the ventilation duct.

WO 2003 098 116 A1 relates to another device for controlling air convection through a cooker hood, based on a measurement of air quality.

EP 3 103 390 A1 discloses a system for protection of a person against contamination of air comprising a portable sensor device with an air pollutant concentration sensor and a physical activity sensor connected to a computation module that determines a personal air pollution indicator of the individual carrying the sensors.

One problem that underlies the present invention comprises providing a system for improved protection of a person in the vicinity of a home appliance for preparing food from air pollutants. The invention solves the given problem through the subject-matter of the enclosed independent claims. The dependent claims describe embodiments of the invention.

Accordingly, an inventive system for protection of a person against contamination of the air in the vicinity of a home appliance for preparing food, especially a cooker, comprises a first sensor for determining a first concentration of a first substance in the air, wherein the first sensor is adapted to be carried on the person; and a processing unit that is adapted to determine, on the basis of said first concentration, an indication of an impact of the first substance on the person's health. The indication may for instance comprise one or several numerical values, each on a given scale, for instance between 1 and 10.

In an embodiment, the system further comprises a second sensor for determining a second concentration of a second substance in the air, wherein the processing unit is adapted to determine the indication on the basis of the first and the second concentration. By combining different measuring spots and/or measurements concerning different potential pollutants, the potential impact on the person's health may be determined more precisely.

In the following, reference is made to a concentration of a substance. In this, the reference may be directed to the first, the second or both concentrations or substances, respectively.

In one embodiment where there are two sensors, the determined concentrations of substances in the air may be put in a relationship to one another in order to determine the indication. A medium concentration of each of a first and a second substance in the air may, in one example, be potentially more harmful than a lower concentration of the first and a higher concentration of the second substance. By relating the determined concentrations to each other, preferably depending on the kind of substances in question, an improved indication of a potential health risk of the person may be provided. For example, if oil is overheated to a point where it smokes, water vapour may better conduct or transport the smoke particles into the respiratory tract of the person. Thus, smoking oil alone may be less hazardous for the person than a combination of smoke and vapour.

This may allow the person to estimate how healthy it is for her to stay where she is and to prepare food the way she does. The indication may lead to improved awareness of the person on how the cooking process can be made less unhealthy. Especially in eastern countries like India or China, where cooking may traditionally involve great heat and a lot of stirring or shaking, a considerable potential for reducing harm through cooking fumes, mist or vapour may be exploited.

The second sensor is usually configured to determine a concentration of a substance different to the one the first sensor is sensitive to. While the first sensor is preferred to be worn or carried on the person, the second sensor may be disposed close to the first sensor or distant to it (or vice versa). For instance, the second sensor may be placed beside or above the home appliance for preparing food, e.g. the cooker, in a ventilation duct leading to or from the home appliance for preparing food or in another toxicologically relevant place. There may be a plurality of second sensors and the second sensors may vary in position and/or the substance they are sensitive to.

It is possible that the processing unit is adapted to determine an indication of an impact of a progression of the substance's concentration over time on the person's health. Injection of substances into the air by the cooker itself, especially if it comprises the burning of gas, or by a cooking ingredient in a cooking dish on the cooker, may vary considerably over time, depending on how the cooking dish or ingredient is treated. For instance, stirring the contents of the cooking dish may increase vaporization. It may also cause a portion of the contents of the cooking disk to be moved to a heat source of the cooker so that a part of the dish in preparation may be overheated or burned. By observing the pollution concentrations over time a more representative indication of the air's harmfulness may be determined.

According to the invention, the first substance is producible by exposing a cooking ingredient of a dish intended for human consumption to heat. The cooking ingredient may especially comprise water and/or a fatty substance. Exposure of water to excessive heat may generate vapour and exposure of oil or fat to excessive heat may produce roasting or combustion products that can be harmful when inhaled. In contrast to general air pollution, which may for instance comprise ozone, CO or CO₂, the source of the cooking air pollutants is generally close by so that the pollutant may be especially harmful for the person. Also, the person may have increased control over air concentrations of food related air pollutants.

One of the substances may comprise a volatile organic compound (VOC), a polycyclic aromatic hydrocarbon (PAH, also polyaromatic hydrocarbon), particulate matter (PM), small molecules or alcohol. A sensor for measuring a VOC concentration may comprise a microelectronic probe with a membrane of a predetermined permeability or a heating for the probe to control selection of the substance measured. A VOC sensor may also be configured to determine a concentration of an alcohol or similar compound. PAH are hydrocarbons, which are organic compounds containing only carbon and hydrogen and which are composed of multiple aromatic rings (organic rings in which the electrons are delocalized). PAHs may be further defined as lacking further branching substituent on these ring structures. A VOC or PAH may especially originate from a burning process, specifically when an organic substance that may for instance be comprised by a food ingredient is burned. PM generally comprises fine dust and soot. Different particle sizes may be considered relevant when determining a PM concentration in the air. For instance, the PM10 or PM2.5 definitions may be applied. The small molecules may comprise H₂, CO, CO₂, NH₃, H₂S, or CH₄. These compounds, too, may be detectable through a semiconductor VOC sensor.

The system may further comprise an interface for receiving additional data related to a substance in the air. This information may comprise pollen or weather data such as wind speed, direction or temperature. Other information can be used to determine the potential harmfulness of the analysed air, too. A general climate or air quality information may help to set the found concentrations in context with what must be considered normal where the cooker is disposed.

According to the invention, an interface is provided for receiving information about a temperature generated by the home appliance for preparing food, e.g. a cooker. The cooker temperature may be taken outside or inside a cooking dish on the cooker to determine the cooking temperature or the temperature that may act upon a portion of food that leaves the cooking dish. It has been found that if the cooking temperature is reduced, possible air contamination may be significantly reduced as well.

The system may further comprise an interface for connection to an air ventilation system that is disposed in the vicinity of the home appliance for preparing food, especially a cooker, wherein the processing unit is adapted to control the air ventilation system in response to said indication, such as to keep said indication within predetermined bounds, especially below a predetermined level. The air ventilation system may be part of a cooker hood and comprise an electric ventilator. The throughput of the air ventilation system may be increased to lower a determined air pollution. In some embodiments the air that passes through the ventilation system may be guided through an extra filter like a HEPA filter in order to decrease a pollutant concentration. This is especially preferred in a case where fresh air is not drawn in from outside a room in which the cooker is disposed (maybe because toxicity of the outside air is considerable). Instead the air is recirculated in the room. Guiding air through a filter may be controlled progressively, for instance such that a smaller portion of the circulating air passes through the filter if pollutant's concentration is low and a bigger portion if the concentration is higher. Air that passes through the filter may be propelled by a dedicated ventilator. Another ventilator may be provided for circulation of air besides the filter. In some embodiments a portion of the passing air may be circulated in the room (at least partly through the filter) and another portion may be exchanged with the outside of the room. The portions may be controlled on the basis of determined concentrations, such as to keep the indication for the impact on the person's health as low as possible or at least under a predetermined threshold. There may also be an output system for an alarm if the determined indication exceeds a predetermined threshold.

The processing unit may be disposed close to one of the sensors or in a remote location. Processing may for instance be done via a remote service like a web-based or a cloud-based service. The processing unit may be adapted to determine a plurality of indications based on associated concentrations. It may for example process data from many cooking sites. Practical experiences made with other data may be utilized to better process present data. Especially in the context of a centralised processing service, availability of a big amount of data may permit to improve processing, for instance by using statistical methods for finding correlations between causes and effects.

According to the invention, the processing unit is adapted to present to the person a suggestion for preparing or cooking food such that the indication may be lowered. One such suggestion may be to use a lower cooking temperature, to switch to an electric cooker, to cover the cooking dish with a lid or sieve or to make sure the contents (especially oil droplets) do not escape the cooking dish, especially do not drop into an open flame of the cooker. In this way, an inexperienced cook may be assisted in learning how to reduce the generation of or exposure to potentially noxious gases or other air polluting substances. The indication of the determined impact on the person's health, e.g. the inverse of the air quality, may be displayed to the person, possibly together with a reference value of what an experienced cook may achieve. The indication may comprise one combined value or a profile of several values in which each value may be based on an associated combination of concentration values.

A device for protection of a person against contamination of the air in the vicinity of a home appliance for preparing food, especially a cooker, not part of the present invention, comprises a first sensor for determining a first concentration of a first substance in the air and a processing unit that is adapted to determine, on the basis of the first concentration, an indication of an impact of the first substance on the person's health, wherein the device is designed to be carried on the person. The device may be small and unobtrusive. It may help to warn of air pollution, may control countermeasures against air pollution, may support a person in learning to reduce air pollution or may collect data on the person's exposure to air pollution during a certain time frame. The device may be an embodiment or a part of the above-described system. Advantages or features mentioned with reference to the system may also be applicable to the device or the below-described method and vice versa.

The device may also be provided with an interface to a second sensor for determining a second concentration of a second substance in the air and the processing unit is adapted to determine said indication on the basis of the first and the second determined concentrations.

In addition, the invention comprises a method for protecting a person against contamination of the air in the vicinity of a home appliance for preparing food, especially a cooker. The method comprises the steps of: determining a first concentration of a first substance in the air, based on a measurement by a first sensor adapted to be carried on the person; and determining by a processing unit, on the basis of the first concentration, an indication of an impact of the first substance on the person's health; receiving information by an interface on a temperature generated by the home appliance for preparing food, wherein said first substance is producible by exposing a cooking ingredient to heat; and presenting to the person an instruction by the processing unit for preparing, especially cooking, food such that the determined indication may be lowered.

The method may be carried out on the processing unit of the above-described system.

The processing unit may especially comprise a programmable microcomputer or microprocessor and the method may exist as a computer program product. In this case the method may be saved on a computer readable media. The program product may especially come in the shape of a so called app or application. It is furthermore preferred that a second concentration of a second substance in the air is determined and the indication is determined on the basis of the first and the second concentration.

The invention has been described with reference to a system and a method. If not stated differently, the features of one claim category are applicable to the other claim categories in analogy.

The invention will now be explained in greater detail with reference to the enclosed figures, in which:
- Fig. 1: shows an embodiment of a system for protection of a person against contamination of the air in the vicinity of a cooker for preparing food;
- Fig. 2: shows a first flow chart how possibly harmful substances may be generated during cooking;
- Fig. 3: shows a second flow chart of an embodiment of a method for protecting a person against contamination of the air in the vicinity of a cooker for preparing food; and
- Fig. 4: shows a time diagram for controlling a ventilation system.

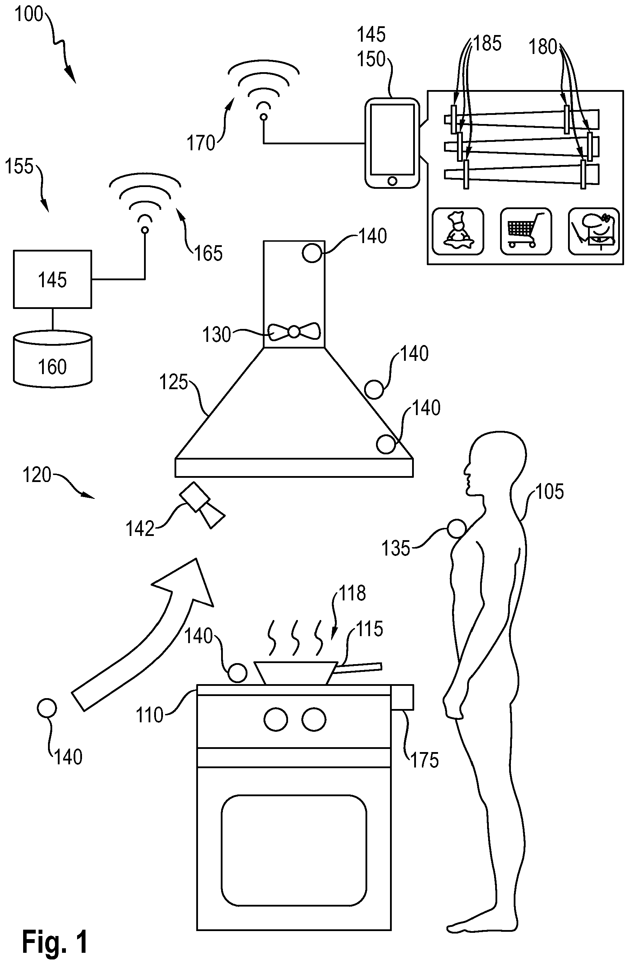

Figure 1 shows a system 100 for protection of a person 105 against contamination of the air in the vicinity of a cooker 110 for preparing food. The cooker 110 may be disposed in an open or a closed space such as a room like a kitchen. On the cooker 110 there may be a cooking tool 115 like a pan or pot in which ingredients 118 are heated to realise a cooking process. The cooker 110 may be powered by gas or electricity and follow an arbitrary physical principle like infrared, convection or induction heating.

In the area of the cooker 110 there may be foreseen an air convection system 120. In the present embodiment the convection system 120 may comprise a cooker hood 125 which is preferred to be disposed above the cooker 110, although a configuration with an air duct besides or below the cooker 110 may also be used. The convection system 120 may comprise a ventilator 130 that may be enclosed in the cooking hood 125. The ventilator 130 may be controllable to assume different turning speeds such as to effect different air mass flows. The ventilator 130 may control an air stream that is removed from the cooker 110 or supplied to the cooker 110, be it from inside or outside a surrounding room.

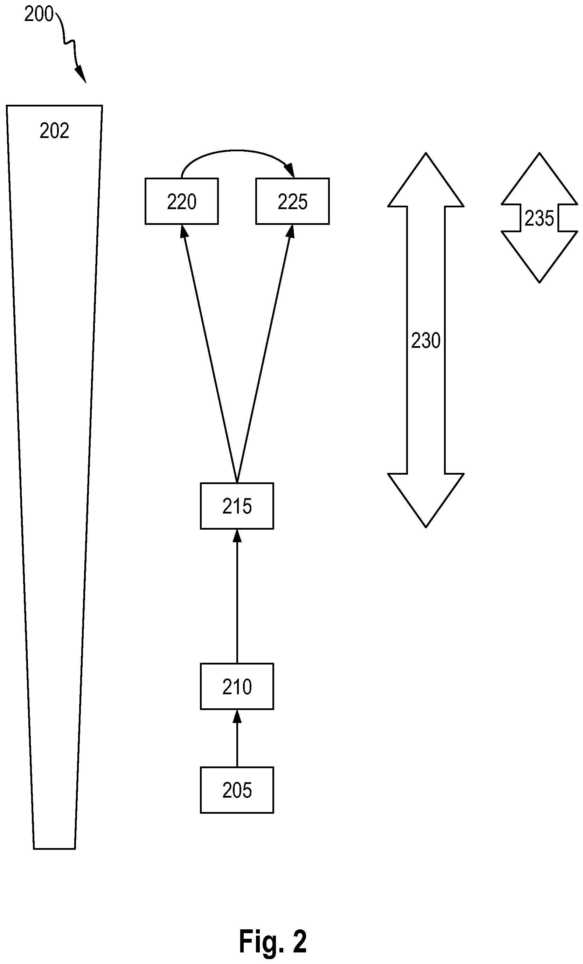

As will be explained in greater detail below with reference to figure 2, the cooking process may spread one or several different substances into the air. Said substances may be harmful to the person 105 if inhaled. Production or dispense of the substances may be related to a cooking process the person 105 performs. Also, the amount of substances the person 105 inhales may be dependent on his activity and precise location and posture to the cooker 110 at a given time. Over a longer period the person 105 may accumulate said substances so that a harmful effect on the person 105 may be caused later.

The system 100 comprises a first sensor 135 that is configured to be carried or worn by the person 105. Optionally, there is also provided a second sensor 140 that may also be disposed on the person 105 or in another place in the general vicinity of the cooker 110. Finally, the system 100 comprises a processing unit 145 that is data linked to the sensors 135 and 140, if applicable. The sensors 135, 140 may each be configured to determine a concentration of a predetermined substance in the surrounding air. The second sensor 140 is preferred to be situated in or next to an air stream that passes the cooker 110. In figure 1, there are several second sensors 140 in different possible positions upstream and downstream of the cooker 110 and the cooking tool 115, as well as, for reference purposes, off the air stream but in the vicinity of the cooker 110. Should the air stream be drawn from outside a room in which the cooker 110 resides there may also be another second sensor 140 disposed outside the room.

Optionally there may also be one or more additional sensors for air related parameters, like a temperature sensor 142 that is configured to determine a temperature of the cooker 110, the cooking tool 115 or an ingredient 120 inside the cooking tool 115. The temperature sensor 142 may be contactless and operate on the principle of detecting infrared radiation. In another embodiment, the temperature or an indication that relates to the temperature may be provided by the cooker 110. The indication may be derived from a determined energy consumption of the cooker 110.

The processing unit 145 is by way of example shown as part of a device 150 that resembles a smartphone, but the device 150 may also come in a housing of any other form factor. In one embodiment, the first sensor 135 and/or at least one of the second sensors 140 are mounted in or at the device 150. A data connection between a first or second sensor 135, 140 and the processing unit 145 is preferred to be wireless and may be based e.g. on Bluetooth, Wi-Fi or ZigBee technology. In another embodiment, one of the sensors 135, 140 may be attached to the processing unit 145 by wire.

In yet another embodiment, the processing unit 145 may be remote to the cooker 110. In figure 1, an alternative processing unit 145 is shown as part of a data or cloud server 155. Data from the sensors 135, 140 may in this case be transferred to the remote processing unit 145 and a determination result may be transferred back also. Both journeys may be conducted at least partly wirelessly. A part of the data connection may comprise an existing wire based or wireless network such as a WLAN, a cell based network or a cable network. A part of the network may also be usable by internet data traffic.

The remote processing unit 145 is preferred to be configured do perform determinations for a plurality of air pollution data. Thus, the data server 155 may be part of a larger number of systems 100. The data server 155 may also comprise storage 160 for storing concentration or air pollution data, preferably with reference to a measurement time and possibly with a relationship to other data such as weather, climate or seasonal data that may affect an overall harmfulness of air to a person 105. Such data may be supplied to the data server 155 via an interface 165 to a data network such as the internet, making use of private or public data or sources or forecast services. In case of the local processing unit 145 in the device 150, said data may be acquired over a similar data network. For this purpose, the device 150 may comprise a data interface 170. In another embodiment, the device 150 may be used to collect air pollution data and relay it to the data service 155, which processes it and sends back a processing result that may then be displayed by the device 150.

The device 150 may comprise data storage similar to storage 160 of data centre 155. Each of the device 150 and/or the sensors 135, 140 may be powered on rechargeable electric accumulators. A cradle 175 for recharging the device 150 or one of the sensors 135, 140 may be provided and the cradle 175 may be disposed in the vicinity of the cooker 110 so that air parameters may still be determined when the actual cooking process is already over and the device 150 or sensor 135, 140 is placed into the cradle 175.

The processing unit 145, be it local or remote, is preferred to be configured to determine, on the basis of measurements of the first sensor 135 and at least one second sensor 140, possibly additional information like a cooking temperature of the temperature sensor 142 or weather or climate information, an indication of the harmfulness of the air that the person 105 next to the cooker 110 is exposed to. The processing 145 unit may comprise a programmable microcomputer running a predetermined computer program product for running a corresponding method.

The provided indication may be presented to the person 105 e.g. optically, acoustically or haptically. According output mechanisms may be comprised by the device 150. In one embodiment, a warning is output if the indication exceeds a predetermined threshold. In yet another embodiment the indication is based on a time series of air pollution data. A cumulative effect of air pollution on the person's 105 health may thus be determined and optionally compared to a threshold value. For instance, the indication may be based on a sliding average over a predetermined time window. For instance, a person 105 that cooks only occasionally may have less risk of becoming sick from air pollution than a professional cook. On the other hand, the professional cook may be more experienced and handle the cooker 110 such that it produces less or less harmful air pollution.

The system 100 may be used to teach or guide the person 105 how to cook with producing reduced air pollution. This may be done through direct feedback, i.e. by giving the person 105 a prompt feedback on how high air pollution or a health impact of the breathable air is, or summary information may be provided to her after a cooking process. Fig. 1 shows example output of data processing software in the upper right corner. For three different substances that may pollute the air in the vicinity of the cooker 100 there are ranges between low harmfulness (on the left) and high harmfulness (on the right). A first indication 180 may be given that relates to the actual local air pollution by each of the substances. Optionally, a second indication 185 may also be given that relates to a different person 105 or different circumstances so that the person 105 can see how relatively harmful his cooking is. Exemplary buttons in the lower section of the depicted screen of the device 150 may relate to functions like "ask an expert"; "buy ingredients" or "find a healthy recipe". The person 105 may thus have an improved guided cooking experience.

In yet one more embodiment the system 100 may also be configured to control the air convection system 120 according to the determined indication. For instance, a wireless or wire bound connection between the device 150 and the ventilator 130 may be used to increase an air stream if an indication for harmful air is determined.

Figure 2 shows a flow chart how possibly harmful substances may be generated during cooking. The shown model may be correct at least in quality for a given cooking process. In present example both a physical flow of substances and a temperature gradient 202 is shown in vertical direction, ranging from lower temperatures at the bottom to higher temperatures at the top, the physical stream starting on the bottom and striving upwards.

In a step 205, a cooking ingredient 118 is added to the cooking process, preferably by putting the ingredient 118 into the cooking tool 115 that is heated on the cooker 110. The shown process can also be passed more rapidly if a portion of the ingredient 118 is exposed to heat directly, i.e. if flung out of the cooking tool 115 and into the heat source of the cooker 110, especially if it comprises an open flame.

While the ingredient 118 gets hotter, water in the ingredient 118 turns to steam that may be formed in a step 210, possibly alongside with oil droplets from a fatty substance of the ingredients 118. This process may be influenced by the nature or kind of ingredient (liquid / solid or meat / vegetable etc.), other ingredients in the cooking dish or other parameters. At this time the average size of particles in air downstream the cooker 110 may increase (i.e. water or oil droplets of > 2 µm). In a step 215, under the influence of still higher temperatures, volatile organic compounds (VOCs) may be formed through hydrolysis or pyrolysis. The generated VOCs may be smelly or odourless and some of them may be harmless while others may not.

Through more pyrolysis reactions at even higher temperatures, polycyclic aromatic hydrocarbon (PAH) may be formed in a step 220. Formation of PAH and carbon-based particles occurs due to chemical reactions (i.e. Diels-Alder reaction) and further pyrolysis of VOCs. PAH can be absorbed by the carbon-based particles and stay in the food as carcinogenic substances. PAH are generally less harmless if inhaled and their harmfulness may largely depend on the partaking substances, the temperatures and the quantities of the exposed substances. Parallel to step 220, carbon particles may be formed in a step 225 on the basis of the VOCs. Also, some of the PAH of step 220 may be absorbed or attached to the carbon particles. The carbon particles (also called particulate matter) may vary in average diameter. Different particle sizes may imply different degrees of harmfulness.

Therefore, by detecting the concentration of VOCs (particularly those corresponding to food burning), or small molecules like H₂, CO, CO₂, NH₃, H₂S, or CH₄, alcohol or fine particles (i.e. carbon based particles) concentrations, the level of food burning can be estimated and used as an indication for the potential harmfulness of the air emerging from the cooking tool or even the food in the cooking tool. The overall health status of each cooking can be estimated by the concentration of both PAH and carbon particles.

A first temperature range 230 indicates a food burning level, that is, a range that is commonly used during cooking food. VOCs and fine particles are usually formed in the first range 230. A second temperature range 235 denotes where cooking may become unhealthy. In the second range 235, PAH and fine particles are also formed.

It is proposed to give a person 105 in the vicinity of a cooker 110 used for preparing food an indication of the current level of potential harmfulness, caused by said substances in the air. This may be facilitated through the above described system 100 or device 150.

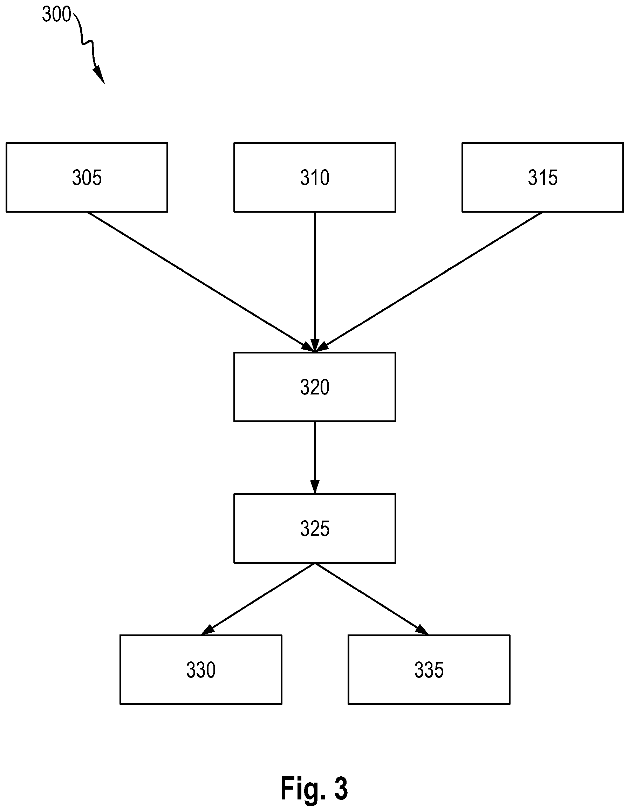

Figure 3 shows a flow diagram of a method 300 for protecting a person 105 against contamination of the air in the vicinity of a cooker 110 for preparing food. The method 300 may especially be carried out, completely or in part, by system 100 or device 150 shown in figure 1.

In a step 305, a first concentration of a first substance in the air is determined. This is preferred to be accomplished using the first sensor 135. In an optional second step 310, a second concentration of a second substance in the air is determined, preferentially using one second sensor 140. The first and second substances may each comprise at least one of a volatile organic compound (VOC), a polycyclic aromatic hydrocarbon (PAH), particulate matter (PM), small molecules or an alcohol. Optionally, more information, for instance on local air pollution, may be gathered in a step 315. This information may comprise more concentration determination using other second sensors; a cooking temperature determined using temperature sensor 142 or external information such as outside air temperature, barometric air pressure or other. The steps 305 through 315 may be carried out concurrently or in an arbitrary sequence. Other additional information may comprise video information from smart electronic appliances in the vicinity of the cooker. For example, this function could help user 105 to identify which cooking process is unhealthy and give recommendations for possible improvement.

In a step 320, the acquired measurements, data and information may optionally be buffered over time. Either way, processing may continue and historic data may be considered as it becomes available.

In a step 325 the data of steps 305 through 315 and/or step 320 is processed to provide one or several indications on the toxicity of the air for the person 105. Determination is preferred to consider that even though a flow of air between the cooking tool 115 and the cooking hood 125 may be significant, the person 105 may stay out of that stream most of the time so that he or she will inhale only small quantities of the polluted air. This consideration may be done on the grounds of concentration data of the first sensor 135 which the person 105 carries around. For best results, the first sensor 135 is preferred to be disposed as close to the mouth and nose of the person 105 as possible.

In a step 330, a behavioural suggestion for the person 105 may be determined, based on the indication determined in step 325, and the suggestions may optionally be provided to the person 105, for instance in an acoustic, written or graphical fashion. The acoustic output may include one or several different warning sounds for different determined circumstances. The output may also come in spoken form, e.g. using text-to-speech technology. The suggestion may be based on additional data such as a recipe that is followed, a setting of the cooker 110 or comparative indication data, for instance an air pollution indication related to a different cook when cooking the same dish. Such a suggestion might e. g. concern using less oil or fat, lowering the cooking temperature, changing the order or the time at which the ingredients 118 are added into the cooking tool 115, when to stay away from the air stream emerging from the cooking tool 115, for instance right after a fatty substance has been added and oil droplet generation is strongest. The indication may be given in the form of a stimulus to the person 105 in a gamification variant of the present technology.

Also, in a step 335, that may be carried out independently of step 330, the air convection system 120, especially the ventilator 130, may be controlled such that the peak or average air pollution for person 105 are reduced. The ventilation system 120 may be switched from recirculation mode to exhaust mode to speed up removal of air pollutants. Ventilation may also be controlled in response to an inside and/or outside temperature, finding a compromise between lowest air pollution at maximum ventilation on the one hand side and most silent and energy efficient air convection at highest pollution values on the other hand side. As pollutant values drop, ventilation may be reduced again.

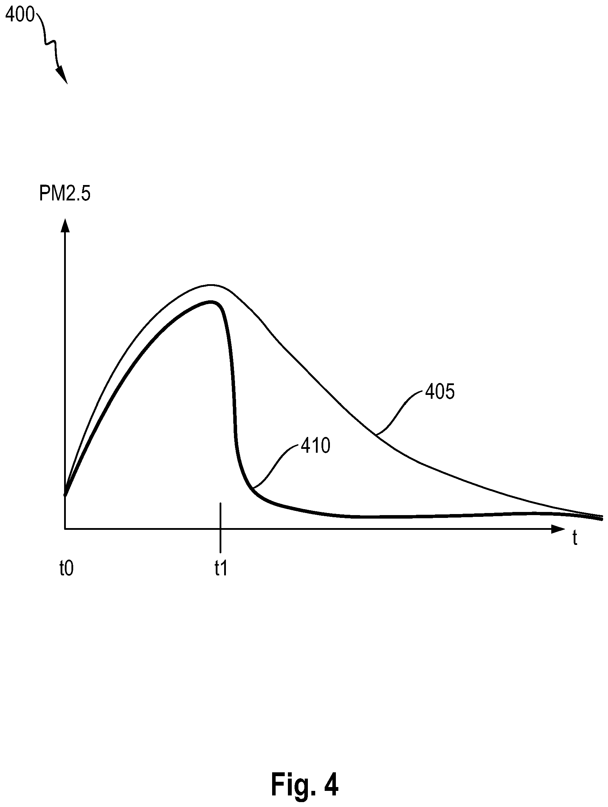

Figure 4 shows a time diagram 400 for controlling an air ventilation system like ventilator 130 in system 100 of figure 1. In a horizontal direction a time and in a vertical direction a concentration of particulate matter in the air around the cooker 110 is displayed, exemplarily expressed in units of PM2.5. In the given example, cooking starts at a point t0 in time and ends at t1. Usually, the ventilator 130 will be shut off at the time cooking is finished because carbon particles in the air cannot be detected by known sensors used for control of ventilator 130. This way, PM2.5 concentrations will be considerable for a longer time, as is expressed by first a trend 405.

However, if control of the air ventilation system 120 is controlled based on actual PM2.5 measurements, concentration of particles in the air can be reduced much quicker than according to the state of the art as a second trend 410 expresses. It is therefore proposed to control air convection based on actual measurements of air pollution, especially a concentration of particulate matter PM of a predetermined average size in the air and control air flow accordingly. This is also explained above with reference to step 335 in method 300. In this, it is preferred that the concentration is measured where harm may be caused, i.e. close to a person 105 in the vicinity of the cooker 110 and especially close to the person's 105 respiratory system. In one embodiment, an extended ventilation time is determined on the basis of the previously determined indication.

The description with regard to the figures is to be understood illustrative, rather than restrictive. Various modifications may be made to the described embodiments without leaving the scope of the invention as set forth in the appended claims.

### Reference Numerals

- 100: system
- 105: person
- 110: cooker
- 115: cooking tool
- 118: ingredient
- 120: air convection or ventilation system
- 125: cooking hood
- 130: ventilator
- 135: first sensor
- 140: second sensor
- 142: temperature sensor
- 145: processing unit
- 150: device
- 155: data server
- 160: storage
- 165: interface
- 170: interface
- 175: cradle
- 180: first indication
- 185: second indication

- 200: flow chart
- 202: temperature
- 205: add cooking ingredient
- 210: formation of steam / oil droplets
- 215: formation of VOCs
- 220: formation of PAH
- 225: formation of carbon particles
- 230: first temperature range: cooking temperatures
- 235: second temperature range: unhealthy range

- 300: method
- 305: determine first concentration
- 310: determine second concentration
- 315: determine additional information
- 320: buffer over time
- 325: determine indication
- 330: determine suggestion
- 335: control ventilation

- 400: time diagram
- 405: first trend
- 410: second trend

## Claims

1. System (100) for protection of a person (105) against contamination of air in a vicinity of a home appliance for preparing food, especially a cooker (110), wherein the system (100) comprises:
- a first sensor (135) for determining a first concentration of a first substance in the air;
- wherein the first sensor (135) is adapted to be carried on the person (105); and
- a processing unit (145) that is adapted to determine, on the basis of the first concentration, an indication of an impact of the first substance on the person's (105) health,
- an interface (165, 170) for receiving information on a temperature generated by the home appliance for preparing food, wherein said first substance is producible by exposing a cooking ingredient (118) to heat, and
- wherein the processing unit (145) is adapted to present to the person (105) an instruction for preparing, especially cooking, food such that the determined indication may be lowered.

2. System (100) according to claim 1, further comprising a second sensor (140) for determining a second concentration of a second substance in the air, wherein the processing unit (145) is adapted to determine the indication on the basis of the first and the second concentration.

3. System (100) according to claim 1 or 2, wherein the processing unit is adapted to determine the indication on the basis of a progression of the first and/or second substance's concentration over time on the person's (105) health.

4. System (100) according to one of the preceding claims, wherein also said second substance is producible by exposing the cooking ingredient (118) to heat.

5. System (100) according to claim 4, wherein the cooking ingredient (118) comprises water and/or a fatty substance.

6. System (100) according to one of claims 4 or 5, wherein the first and/or second substance comprises at least one of a volatile organic compound, a polycyclic aromatic hydrocarbon, particulate matter, small molecules or alcohol.

7. System (100) according to one of the preceding claims, further comprising an interface (165, 170) for receiving additional data related to a substance in the air.

8. System (100) according to one of the preceding claims, further comprising an interface (165, 170) for connection to an air ventilation system (120) that is disposed in the vicinity of the home appliance for preparing food, especially the cooker (110), wherein the processing unit (145) is adapted to control the air ventilation system (120) in response to said indication, such as to keep the indication below a predetermined level.

9. System (100) according to one of the preceding claims, wherein the processing (145) unit is adapted to determine a plurality of indications based on associated concentrations of substances in the air.

10. Method (300) for protecting a person (105) against contamination of air in a vicinity of a home appliance for preparing food, especially a cooker (110), wherein the method comprises steps of:
- determining (305) a first concentration of a first substance in the air, based on a measurement by a first sensor (135) adapted to be carried on the person (105);
- determining (325) by a processing unit (145), on the basis of the first concentration, an indication of an impact of the first substance on the person's (105) health,
- receiving information by an interface (165,170) on a temperature generated by the home appliance for preparing food, wherein said first substance is producible by exposing a cooking ingredient (118) to heat, and
- presenting to the person (105) an instruction by the processing unit (145) for preparing, especially cooking, food such that the determined indication may be lowered.

## Patentansprüche

1. System (100) zum Schutz einer Person (105) gegen Luftverschmutzung in der Nähe eines Haushaltsgeräts zur Nahrungsmittelzubereitung, insbesondere eines Kochgeräts (110), wobei das System (100) umfasst:
- einen ersten Sensor (135), um eine erste Konzentration einer ersten Substanz in der Luft zu bestimmen;
- wobei der erste Sensor (135) dafür ausgelegt ist, an der Person (105) getragen zu werden; und
- eine Verarbeitungseinheit (145), die dafür ausgelegt ist, basierend auf der ersten Konzentration eine Angabe bezüglich einer Auswirkung der ersten Substanz auf die Gesundheit der Person (105) zu bestimmen;
- eine Schnittstelle (165, 170) zum Empfangen von Informationen über eine Temperatur, die vom Haushaltsgerät zur Nahrungsmittelzubereitung erzeugt wird, wobei die erste Substanz durch Aussetzen einer Kochzutat (118) gegenüber Wärme erzeugbar ist, und
- wobei die Verarbeitungseinheit (145) dafür ausgelegt ist, der Person (105) eine Anweisung zur Zubereitung, insbesondere zum Kochen, von Nahrungsmitteln zu präsentieren, so dass die festgelegte Angabe abgesenkt werden kann.

2. System (100) nach Anspruch 1, ferner umfassend einen zweiten Sensor (140) zum Bestimmen einer zweiten Konzentration einer zweiten Substanz in der Luft, wobei die Verarbeitungseinheit (145) dafür ausgelegt ist, die Angabe basierend auf der ersten und der zweiten Konzentration zu bestimmen.

3. System (100) nach Anspruch 1 oder 2, wobei die Verarbeitungseinheit dafür ausgelegt ist, die Angabe basierend auf dem Verlauf der Konzentration der ersten und/oder der zweiten Substanz über die Zeit zu der Gesundheit der Person (105) zu bestimmen.

4. System (100) nach einem der vorhergehenden Ansprüche, wobei auch die zweite Substanz durch Aussetzen der Kochzutat (118) gegenüber Wärme erzeugbar ist.

5. System (100) nach Anspruch 4, wobei die Kochzutat (118) Wasser und/oder eine Fettsubstanz umfasst.

6. System (100) nach einem der Ansprüche 4 oder 5, wobei die erste und/oder die zweite Substanz mindestens eines von einer flüchtigen organischen Verbindung, einem polyzyklischen aromatischen Kohlenwasserstoff, Feststoffteilchen, kleine Moleküle oder Alkohol umfasst.

7. System (100) nach einem der vorhergehenden Ansprüche, ferner umfassend eine Schnittstelle (165, 170) zum Empfangen von zusätzlichen Daten in Bezug auf eine Substanz in der Luft.

8. System (100) nach einem der vorhergehenden Ansprüche, ferner umfassend eine Schnittstelle (165, 170) zur Verbindung mit einem Belüftungssystem (120), das in der Nähe des Haushaltsgerätes zur Nahrungsmittelzubereitung, insbesondere des Kochgeräts (110), angeordnet ist, wobei die Verarbeitungseinheit (145) dafür ausgelegt ist, das Belüftungssystem (120) in Reaktion auf die Angabe so zu steuern, dass die Angabe unter einem vorbestimmten Pegel gehalten wird.

9. System (100) nach einem der vorhergehenden Ansprüche, wobei die Verarbeitungseinheit (145) dafür ausgelegt ist, eine Vielzahl von Angaben basierend auf zugehörigen Konzentrationen von Substanzen in der Luft zu bestimmen.

10. Verfahren (300) zum Schutz einer Person (105) gegen Luftverschmutzung in der Nähe eines Haushaltsgeräts zur Nahrungsmittelzubereitung, insbesondere eines Kochgeräts (110), wobei das Verfahren die folgenden Schritte umfasst:
- Bestimmen (305) einer ersten Konzentration einer ersten Substanz in der Luft basierend auf einer Messung durch einen ersten Sensor (135), der dafür ausgelegt ist, an der Person (105) getragen zu werden;
- Bestimmen (325), durch eine Verarbeitungseinheit (145), einer Angabe bezüglich einer Auswirkung der ersten Substanz auf die Gesundheit der Person (105) basierend auf der ersten Konzentration;
- Empfangen von Informationen, über eine Schnittstelle (165, 170), über eine Temperatur, die von dem Haushaltsgerät zur Nahrungsmittelzubereitung erzeugt wird, wobei die erste Substanz durch Aussetzen einer Kochzutat (118) gegenüber Wärme erzeugbar ist; und
- Präsentieren, durch die Verarbeitungseinheit (145), einer Anweisung zur Zubereitung, insbesondere zum Kochen, von Nahrungsmitteln für die Person, so dass die bestimmte Angabe abgesenkt werden kann.

## Revendications

1. Système (100) pour la protection d'une personne (105) contre la contamination de l'air dans le voisinage d'un appareil ménager pour la préparation d'aliments, en particulier une cuisinière (110), dans lequel le système (100) comprend :
- un premier capteur (135) pour déterminer une première concentration d'une première substance dans l'air ;
- dans lequel le premier capteur (135) est adapté afin d'être porté sur la personne (105) ; et
- une unité de traitement (145) adaptée afin d'établir, sur la base de la première concentration, une indication de l'impact de la première substance sur la santé de la personne (105),
- une interface (165, 170) pour la réception d'informations sur une température générée par l'appareil ménager pour la préparation d'aliments, dans lequel ladite première substance peut être produite en exposant un ingrédient à cuire (118) à la chaleur, et
- dans lequel l'unité de traitement (145) est adaptée afin de présenter à la personne (105) une instruction afin de préparer, en particulier de cuire, les aliments de sorte à pouvoir réduire l'indication établie.

2. Système (100) selon la revendication 1, comprenant en outre un deuxième capteur (140) pour déterminer une deuxième concentration d'une deuxième substance dans l'air, dans lequel l'unité de traitement (145) est adaptée afin d'établir l'indication sur la base de la première et de la deuxième concentration.

3. Système (100) selon la revendication 1 ou 2, dans lequel l'unité de traitement est adaptée afin d'établir l'indication sur la base d'une progression de la concentration de la première et/ou de la deuxième substance dans le temps sur la santé de la personne (105).

4. Système (100) selon l'une des revendications précédentes, dans lequel ladite deuxième substance peut également être produite en exposant l'ingrédient à cuire (118) à la chaleur.

5. Système (100) selon la revendication 4, dans lequel l'ingrédient à cuire (118) comprend de l'eau et/ou une substance graisseuse.

6. Système (100) selon l'une des revendications 4 ou 5, dans lequel la première et/ou la deuxième substance comprend au moins un composé organique volatil, un hydrocarbure aromatique polycyclique, des particules, de petites molécules ou de l'alcool.

7. Système (100) selon l'une des revendications précédentes, comprenant en outre une interface (165, 170) pour la réception de données supplémentaires liées à une substance présente dans l'air.

8. Système (100) selon l'une des revendications précédentes, comprenant en outre une interface (165, 170) pour la connexion à un système de ventilation d'air (120) disposé dans le voisinage d'un appareil ménager pour la préparation d'aliments, en particulier de la cuisinière (110), dans lequel l'unité de traitement (145) est adaptée afin de commander le système de ventilation d'air (120) en réponse à ladite indication, de sorte à maintenir l'indication en-deçà d'un niveau prédéterminé.

9. Système (100) selon l'une des revendications précédentes, dans lequel l'unité de traitement (145) est adaptée afin d'établir une pluralité d'indications fondées sur des concentrations associées de substances dans l'air.

10. Procédé (300) pour la protection d'une personne (105) contre la contamination de l'air dans le voisinage d'un appareil ménager pour la préparation d'aliments, en particulier une cuisinière (110), dans lequel le procédé comprend les étapes suivantes :
- détermination (305) d'une première concentration d'une première substance dans l'air, sur la base d'une mesure effectuée par un premier capteur (135) adapté afin d'être porté sur la personne (105) ;
- détermination (305) par une unité de traitement (145), sur la base de la première concentration, une indication de l'impact de la première substance sur la santé de la personne (105),
- réception par une interface (165, 170) d'informations sur une température générée par l'appareil ménager pour la préparation d'aliments, dans lequel ladite première substance peut être produite en exposant un ingrédient à cuire (118) à la chaleur, et
- présentation à la personne (105) par l'unité de traitement (145) d'une instruction afin de préparer, en particulier de cuire, les aliments de sorte à pouvoir réduire l'indication établie.
